(19) Europäisches Patentamt
European Patent Office
Office européen des brevets

(11) Veröffentlichungsnummer: **0 394 983**
**A2**

(12) **EUROPÄISCHE PATENTANMELDUNG**

(21) Anmeldenummer: **90107813.9**

(51) Int. Cl.5: **C07H 15/252, A61K 31/70**

(22) Anmeldetag: **25.04.90**

(30) Priorität: **26.04.89 DE 3913743**

(43) Veröffentlichungstag der Anmeldung: **31.10.90 Patentblatt 90/44**

(84) Benannte Vertragsstaaten: **AT BE CH DE DK ES FR GB GR IT LI LU NL SE**

(71) Anmelder: **BEHRINGWERKE Aktiengesellschaft**
**Postfach 1140**
**D-3550 Marburg 1(DE)**

(72) Erfinder: **Hermentin, Peter, Dr.**
**Salzköppel 9**
**D-3550 Marburg(DE)**
Erfinder: **Raab, Ernst**
**Weidenbrunkel 13**
**D-3550 Marburg(DE)**
Erfinder: **Hoffmann, Dieter, Dr.**
**Im Stetefeld 6**
**D-3551 Lahntal(DE)**
Erfinder: **Kraemer, Hans Peter, Dr., Dr.**
**Birkenweg 16**
**D-3550 Marburg(DE)**

(74) Vertreter: **Klein, Otto, Dr. et al**
**Hoechst AG Zentrale Patentabteilung**
**Postfach 80 03 20**
**D-6230 Frankfurt am Main 80(DE)**

(54) **Zytostatisch wirksame rhodomycinderivate.**

(57) Die Erfindung betrifft neue, zytostatisch wirksame Anthracyclinderivate der Formel I

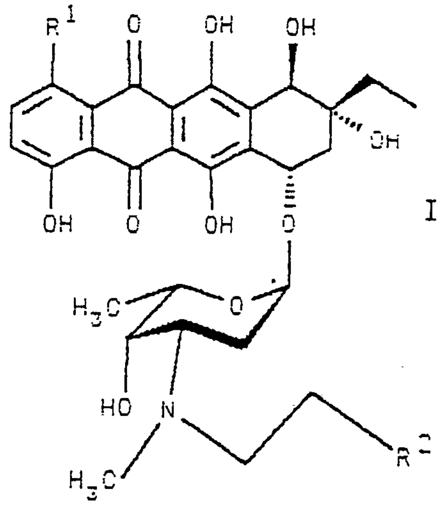

I

und deren physiologisch unbedenklichen Salze, wobei $R^1$ Wasserstoff oder eine Hydroxygruppe, $R^2$ Cl, Br, $OCOR^x$, $NR^aR^b$, $NR^a-(CH_2)_n-NR^aR^b$, $NR^a-NR^a-CO-(CH_2)_y-CO-NR^a-NR^aR^b$ oder $NR^a-CHR^3R^4$ ist, wobei $R^x$ $C_1$-$C_6$-Alkyl, Phenyl oder Benzyl, n = 0-12 und y = 0-10 ist, wobei $R^a$ und $R^b$ jeweils Wasserstoff, $C_1$-$C_4$-Alkyl oder Benzyl sein kann, und wobei $R^3$ Wasserstoff oder eine Carboxylgruppe und $R^4$ der Rest einer L-Aminosäure oder eines biogenen Amins ist, ein Verfahren zu ihrer Herstellung und ihre Verwendung in

EP 0 394 983 A2

einem Arzneimittel.

## Zytostatisch wirksame Rhodomycinderivate

Die Erfindung betrifft neue zytostatisch wirksame Anthracyclinderivate der Formel I und deren physiologisch unbedenklichen Salze, Verfahren zu ihrer Herstellung sowie ihre Verwendung als Arzneimittel.

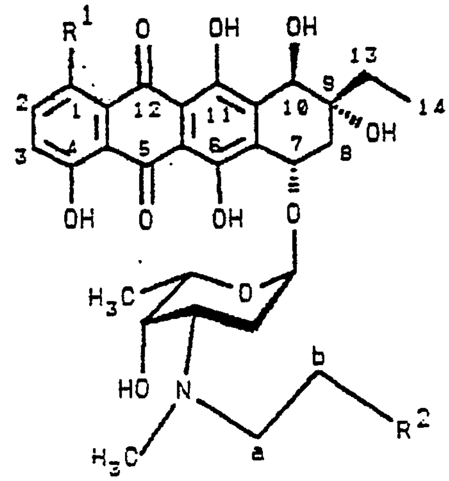

Für Formel I gilt:
$R^1$ ist Wasserstoff oder eine Hydorxygruppe und
$R^2$ ist Cl, Br oder $OCOR^x$, wobei $R^x$ $C_1$-$C_6$-Alkyl, verzweigt oder unverzweigt, Phenyl oder Benzyl ist, oder
y = 0-10, bevorzugt y = 0-5 ist, wobei $R^a$ und $R^b$ jeweils Wasserstoff, $C_1$-$C_4$-Alkyl oder Benzyl sein können, oder
$R^2$ ist $NR^a$-$CHR^3$-$CH_2$-$R^4$,
wobei $R^a$ die genannte Bedeutung hat und $R^3$ Wasserstoff oder eine Carboxylgruppe und $R^4$ den Rest einer L-Aminosäure oder eines biogenen Amins, bevorzugt den Rest einer aromatischen L-Aminosäure oder eines aromatischen Amins Tyramin, Tryptamin, Histamin, Dopamin oder 5-Hydroxydopamin und besonders bevorzugt einen Histaminyl-Rest darstellt, wobei die L-Aminosäure durch $R^2$ mit $R^3$ = Carboxyl und das biogene Amin durch $R^2$ mit $R^3$ = Wasserstoff definiert ist, oder
$R^2$ ist $OCOR^x$, wobei $R^x$ $C_1$-$C_6$-Alkyl, verzweigt oder unverzweigt, Phenyl oder Benzyl darstellt.

Von den durch chirurgische bzw. strahlentherapeutische Maßnahmen nicht mehr heilbaren malignen Tumoren sind derzeit etwa 7 bis 10 % durch Chemotherapie heilbar. Insbesondere weisen schnell proliferierende Tumoren, wie z. B. die akute lymphatische Leukämie des Kindes, der Morbus Hodgkin, Hodentumoren und Wilmstumoren eine hohe Sensitivität gegen Chemotherapie mit Remissionsraten von 60 bis 90 % auf.

Weiterhin kann durch Chemotherapie bei ca. 30 % aller Tumorpatienten eine Verlangsamung des Tumorwachstums bzw. eine partielle oder komplette Remission der Tumorerkrankungen erreicht werden. Nach unterschiedlich langen Zeitintervallen tritt jedoch auch bei Fortsetzung der Chemotherapie ein Rezidiv des Tumors auf, der dann zumeist gegen die zunächst erfolgreiche Chemotherapie resistent ist. Diese unter Chemotherapie auftretende sogenannte "sekundäre Resistenz" begrenzt dann die Möglichkeit einer weiteren chemotherapeutischen Beeinflussung des Tumors. Zu dieser Gruppe von Tumoren gehören die Mamma- und Ovarialtumoren, die kleinzelligen Lungenkarzinome sowie ein Teil der Lymphome.

Viele maligne Erkrankungen lassen sich derzeit jedoch durch Chemotherapie nicht oder nur marginal beeinflussen, so daß in diesen Fällen von einer primären Resistenz des Tumors gegen die heute bekannten Substanzen ausgegangen werden muß. In diese Gruppe der Tumoren gehören die nicht kleinzelligen Lungentumoren sowie der große Teil der Gastrointestinaltumoren. Betrachtet man diese Gruppe der primär resistenten Tumoren unter dem Aspekt ihrer Proliferationsgeschwindigkeit, so fällt auf, daß gerade die schwer oder nicht durch Chemotherapie beeinflußbaren Tumoren eine besonders langsame Proliferationsrate aufweisen.

Mit höchster Priorität gilt es daher nach neuen Substanzen zu suchen, die gerade bei langsam proliferierenden, primär resistenten Humantumoren wirksam sind. Auch sollten zukünftige Chemotherapeutika möglichst keine Kreuzresistenz zu bereits bekannten Substanzen aufweisen, um mit Aussicht auf Erfolg

auch bei vorliegender sekundärer Resistenz gegen bereits etablierte Substanzen einsetzbar zu sein.

Aus der Klasse der Anthracycline besitzen besonders Doxorubicin (Adriamycin), Epirubicin oder Daunomycin bei einer Vielzahl von Tumorerkrankungen therapeutische Wirksamkeit. Jedoch wird nach mehrmaliger Behandlung eine deutliche Verringerung der antitumoralen Wirksamkeit bis hin zur Wirkungslosigkeit beobachtet, bedingt durch eine sich aufbauende "sekundäre Resistenz" der Tumorzellen gegen das Anthracyclin.

Bei der tumortherapeutischen Verwendung dieser bekannten Anthracycline liegt ein weiteres wesentliches Problem darin, daß diese Verbindungen neben der gewünschten zytostatischen Wirksamkeit auch unerwünschte Nebenwirkungen aufweisen, wie beispielsweise eine hämatologische oder cardiale Toxizität.

Die tumortherapeutische Wirksamkeit von Daunomycin und Adriamycin wird vor allem auf die Fähigkeit dieser Anthracycline zur Interkalation in die DNA zurückgeführt, was das Wachstum der Tumorzelle unterbindet und den Zelltod herbeiführt.

Ausgehend von diesem Stand der Technik ist es Aufgabe der vorliegenden Erfindung, neue Anthracyclinderivate zu schaffen, die möglichst nicht kreuzresistent gegenüber Adriamycin sind und die sich durch ein neues Wirkungsspektrum und eine im Vergleich zu Adriamycin geringere Toxizität auszeichnen und somit vorteilhafte Anwendung in der Tumortherapie finden können.

Zu diesem Zweck wurde bereits vorgeschlagen, Rhodosaminyl-Anthracyclinone der Formel I zu synthetisieren, in denen $R^1$ die genannte Bedeutung hat und $R^2$ $C_1$-$C_6$-Alkyl (Hermentin et al., DE 3641833 A1) oder eine Hydroxygruppe ist.

Überraschenderweise wurde nun gefunden, daß sich das von uns (Hermentin et al., DE 3641833 A1) beschriebene Anthracyclin 7-0- (3′N-Methyl-α-L-daunosaminyl)-β-rnodomycinon durch Umsetzung mit Chloracetaldehyd in eine Verbindung der Formel I mit $R^1$ = H und $R^2$ = Cl überführen läßt. Von dieser Verbindung der Formel I mit $R^2$ = Cl konnte gezeigt werden, daß sie unter schwach sauren Bedingungen überraschenderweise relativ stabil ist, daß jedoch der Rest $R^2$ = Cl unter basischen Bedingungen leicht ausgetauscht werden kann. Beispielsweise führt wäßrige alkalische Hydrolyse der genannten Verbindungen zur Substitution des Chloratoms durch eine Hydroxygruppe und somit zu einem Anthracyclin, dessen Synthese von uns bereits vorgeschlagen worden ist.

Ferner konnte gezeigt werden, daß sich die genannte Verbindung der Formel I mit $R^2$ = Cl als Schlüsselverbindung für die Umsetzung mit Aminogruppen-haltigen Verbindungen eignet und daß sich die so gewonnenen Derivate gegenüber den Anthracyclinen des Standes der Technik in ihrer Wasserlöslichkeit und/oder Reaktivität und/oder Toxizität unterscheiden.

Beispielsweise wurde gefunden, daß sich das Chloratom durch eine Dimethylaminogruppe substituieren läßt und daß die Verbindung mit R = $^1$ H und $R^2$ = $N(CH_3)_2$ amphotere Eigenschaften besitzt und seine Wasserlöslichkeit im Vergleich zu den Anthracyclinen des Standes der Technik erhöht ist.

Ferner wurde gefunden, daß sich das Chloratom durch Umsetzung mit N,N′-Dimethyl-ethylendiamin zu einer Verbindung mit $R^1$ = H und $R^2$ = $N(CH_3)$-$CH_2$-$CH_2$-NH-$CH_3$ umsetzen läßt.

Weiterhin wurde gefunden, daß sich eine Verbindung der Formel I mit $R^1$ = H und $R^2$ = Cl auch mit Histamin umsetzen läßt, wobei eine Verbindung der Formel I erhalten wird, in welcher $R^2$ ein Histaminyl-Rest ist.

Weiterhin wurde gefunden, daß sich eine Verbindung der Formel I mit $R^1$ = H und $R^2$ = Cl auch mit Natriumacetat umsetzen läßt, wobei eine Verbindung der Formel I erhalten wird, in welcher $R^2$ $OCOCH_3$ ist.

Gegenstand der Erfindung sind daher Verbindungen der Formel I mit den angegebenen Bedeutungen der Variablen.

Bevorzugte Bedeutungen sind:

$R^2$ ist Cl oder Br, bevorzugt jedoch Cl;
$R^2$ ist $NR^aR^b$;
$R^2$ ist $NR^a(-CH_2)_n-NR^aR^b$;
$R^2$ ist $NR^a-NR^a-CO-(CH_2)_y-CO-NR^a-NR^aR^b$;
$R^2$ ist $NR^a-CHR^3-CH_2-R^4$, wobei die Verbindungen, in welchen $R^3$ Wasserstoff ist, bevorzugt sind;
$R^4$ ist der Rest einer aromatischen L-Aminosäure oder eines aromatischen biogenen Amins;
$R^4$ ist der Rest des biogenen Amins Tyramin, Tryptamin, Histamin, Dopamin oder 5-Hydroxydopamin und bevorzugt der Rest des biogenen Amins Histamin;
$R^2$ ist $OCOR^x$, wobei $R^x$ = $CH_3$ bevorzugt ist.

Das Verfahren zur Herstellung der neuen erfindungsgemäßen zytostatisch wirksamen Anthracyclinderivate der Formel I besteht darin, daß man eine Verbindung der Formel II,

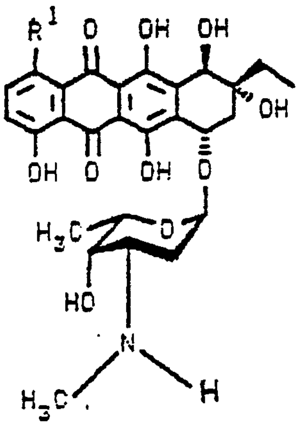

II

(Hermentin et al., DE 3641833 Al), in welcher $R^1$ die genannte Bedeutung hat, durch Umsetzung mit Chloracetaldehyd oder Bromacetaldehyd in Gegenwart von Natriumcyanoborhydrid in eine Verbindung der Formel I überführt, in welcher $R^1$ die genannte Bedeutung hat und $R^2$ = Cl ist, worauf man diese Verbindung mit einer Verbindung der Formel $MeOCOR^x$, worin $R^x$ die genannte Bedeutung hat und Me für ein Alkaliion steht, oder mit einer Verbindung der Formel $HNR^aR^b$ oder $HR^aN-(CH_2)_n-NR^aR^b$ oder $HNR^a-NR^a-CO-(CH_2)_y-CO-NR^a-NR^aR^b$, worin n, $R^a$, $R^b$ und Y die genannte Bedeutung haben, oder mit einem biogenen Amin oder einer Aminosäure in einem geeigneten Lösungsmittel, wie beispielsweise Dimethylformamid, bei einer Temperatur zwischen beispielsweise 0 °C und der Siedetemperatur des Lösungsmittels zur Reaktion bringt und die so erhaltene Verbindung der Formel I isoliert und aufreinigt.

Die nach dem erfindungsgemäßen Verfahren erhaltenen neuen Anthracycl inderivate zeichnen sich durch zytostatische Aktivität aus, und sie können daher zusammen mit den üblichen pharmazeutischen Konfektionierungs- und/oder Verdünnungsmitteln zu Arzneimitteln verarbeitet werden, die in der Krebstherapie Anwendung finden. Die Dosierungs- und Anwendungsweise entspricht dabei im wesentlichen derjenigen für die bekannten Substanzen Adriamycin, Daunomycin, Aclacinomycin, 4′-epi-Adriamycin, 4′-Methoxyadriamycin oder 4′-Desoxyadriamycin.

Die solchermaßen hergestellten Arzneimittel können zusätzlich noch andere Wirkstoffe enthalten, sofern diese zusammen mit den erfindungsgemäßen Verbindungen keine unerwünschten Nebenwirkungen zeigen.

Die zytostatische Wirksamkeit der erfindungsgemäßen Verbindungen wurde anhand von L1210 Leukämiezellen der Maus getestet. Hierzu wurde die Koloniebildung von L1210 Leukämiezellen in Agarplatten herangezogen. Diese Methode dient zum Nachweis des Einflusses der Testsubstanzen auf das Wachstumsverhalten der Zellen über 1 Stunde oder über mehrere Generationen. Bei einer Zellzykluszeit von 10-12 Stunden werden dabei in der Testzeit von 7 Tagen etwa 14 aufeinanderfolgende Generationen beobachtet. Die erfindungsgemäßen zytostatisch wirksamen Substanzen bewirken in diesem Test eine Reduktion der zu beobachtenden Koloniezahl gegenüber einer unbehandelten Kontrollprobe.

Einzelheiten des angewandten Testverfahrens ergeben sich aus den nachfolgenden Beschreibungen der Verfahrensweisen zur Ermittlung der Koloniebildung.

Zur Erläuterung des erfindungsgemäßen Herstellungsverfahrens werden nachfolgend Beispiele 1 bis 5 aufgeführt, in denen bevorzugte erfindungsgemäße Verbindungen nach den beanspruchten Verfahren hergestellt wurden.

Charakterisierung von Verbindungen der Formel I

Der Verlauf der Reaktionen sowie die resultierenden Verbindungen wurden dünnschichtchromatographisch oder mit der HPLC-Technik untersucht. Dünnschichtchromatographie erfolgte, sofern nicht anders vermerkt, auf Kieselgel-Fertigplatten (Merck). Säulenchromatographie erfolgte über Kieselgel 60 (Merck) der Korngröße 0.040-0.063 mm. Die Ausbeuten sind nicht optimiert.

Für die Dünnschicht- und Säulenchromatographie wurden folgende Laufmittelgemische verwendet (Angaben jeweils in Volumenprozent):

| Laufmittelgemische | A | B | C |
|---|---|---|---|
| Zusammensetzung | | | |
| Chloroform (%) | 70 | 89 | 77 |
| Methanol (%) | 18 | 7.4 | 14 |
| Essigsäure (%) | 8.5 | 3 | 7 |
| Wasser (%) | 3.5 | 0.6 | 2 |

Die Strukturen der hergestellen Verbindungen wurden mittels $^1$H-NMR- sowie MS-Spektroskopie ermittelt.

## BEISPIELE

### Beispiel 1:

7-0-(3′-N-Chlorethyl-3′-N-methyl-α-L-daunosaminyl)-β-rhodomycinon (Verbindung 1)

Eine Lösung von 7-0-(3′-N-methyl-α-L-daunosaminyl)-β-rhodomycinon (100 mg = 0,19 mmol) in Acetonitril (120 ml) wurde nacheinander mit Essigsäure (50 µl, ca. 4,6 equiv.), Chloracetaldehyd (130 µl einer 50-proz. (w/v) wäßrigen Lösung) und Natriumcyanoborhydrid (75 mg) versetzt und 1 h bei Raumtemperatur im Dunkeln gerührt. Dann wurde die Reaktionslösung mit Ethylacetat versetzt und überschüssiges Natriumcyanoborhydrid mittels wäßriger Kochsalzlösung extrahiert und die wäßrige Phase viermal mit Ethylacetat rückextrahiert. Dann wurden die vereinigten organischen Phasen mittels gesättigter Kochsalzlösung rückextrahiert, mit Natriumsulfat getrocknet und am Rotationsverdampfer im Vakuum zur Trockne eingeengt. Das so erhaltene Rohprodukt (95 mg) wurde wegen der hohen Zersetzlichkeit des Produktes auf Kieselgel ohne chromatographische Reinigung für weiterführende Umsetzungen eingesetzt. Zur Charakterisierung des Chlorethyl-Derivates wurde ein 150 mg-Ansatz nach der Aufarbeitung über 15 g Kieselgel im Laufmittelgemisch B chromatographiert, wegen beobachteter Zersetzung im Laufmittelgemisch C rechromato graphiert und schließlich zum dritten in Chloroform/Ethanol (10/1) aufgereinigt. Die analytischen Daten stammen von dem hierbei erhaltenen Reinprodukt (5,2 mg) (Laufmittelgemisch B: $R_F$ 0,30).
MS-FAB $(M+H^+)$ m/e = 592
MS-FAB $(M+H^+-Cl^-)$ m/e = 556
$^1$H NMR (300 MHz, $CDCl_3$) δ 1.12 (t, 3H, $J_{13,14}$ = 7.4 Hz, Me-14), 1.41 (d, 3H, $J_{5',6'}$ = 6.4 Hz, Me-6′), 1.7-2.0 (m, 4H, $CH_2$-2′ und $CH_2$-13), 2.12 (dd, 1H, $J_{7,8a}$ = 4 Hz, $J_{8a,8b}$ = 15 Hz, H-8a), 2.25 (d, 1H, $J_{8a,8b}$ = 15 Hz, H-8b), 2.26 (s, 3H, N-$CH_3$), 2.56 (m, 1H, H-3′), 2.73 (m, 1H, H-a), 2.81 (m, 1H, H-a′), 3.51 (bt, 2H, $J_{a,b}$ = 6.5 Hz, $CH_2$-b), 3.68 (bs, 1H, H-4′), 4.04 (s, 1H, OH-9), 4.09 (q, 1H, $J_{5',6'}$ = 6.1 Hz, H-5′), 4.91 (s, 1H, H-10), 5.16 (m, 1H, H-7), 5.53 (bd, 1H, $J_{1',2'}$ = 2.5 Hz, H-1′), 7.34 (dd, 1H, $J_{1,3}$ = 1 Hz, $J_{2,3}$ = 8.4 Hz, H-3), 7.73 (t, 1H, $J_{1,2}$ = $J_{2,3}$ = 8 Hz, H-2), 7.89 (dd, 1H, $J_{1,2}$ = 7.5 Hz, $J_{1,3}$ = 1 Hz, H-1), 12.16 (bs, 1H, OH-4), 12.85 (bs, 1H, OH-6), 13.61 (bs, 1H, OH-11).

### Beispiel 2:

7-0-(3′-N-Dimethylaminoethyl-3′-N-methyl-α-L-,daunosaminyl)-β-rhodomycinon (Verbindung 2)

Eine jung von Verbindung 1 (12 mg 0,020 mmol) in Ethanol (2 ml) wurde mit 100 µl einer 40-proz. wäßrigen Lösung von Dimethylamin (w/v) versetzt und 16 h bei Raumtemperatur im Dunkeln gerührt. Dann wurde am Rotationsverdampfer zur Trockne eingeengt und das Reaktionsgemisch über 5 g Kieselgel im Laufmittelgemisch A getrennt ($R_F$ 0,2). Die vereinigten Fraktionen wurden zur Phasentrennung mit Wasser versetzt, mit 10-proz. (w/v) Natronlauge auf pH 7 gebracht und danach durch Zusatz gesättigter wäßriger Natriumhydrogencarbonat-Lösung auf pH 9 eingestellt. Dann wurden die Phasen im Scheidetrichter getrennt, die wäßrige Phase mehrmals mit Chloroform extrahiert und die vereinigten organischen Phaser am

Rotationsverdampfer zur Trockne eingeengt.
Ausbeute: 7 mg (0,012 mmol) = 60 %
MS-FAB $(M+H^+)$ m/e = 601
$^1H$ NMR (300 MHz, $CDCl_3$) $\delta$ 1.12 (t, 3H, $J_{13,14}$ = 7.4 Hz, Me-14), 1.39 (d, 3H, $J_{5',6'}$ = 6.4 Hz, Me-6'), 1.7-1.9 (m, 4H, $CH_2$-2' und $CH_2$-13), 2.10 (dd, 1H, $J_{7,8a}$ = 4 Hz, $J_{8a,8b}$ = 15 Hz, H-8a), 2.20 (d, 1H, $J_{8a,8b}$ = 15 Hz, H-8b), 2.22 (s, 3H, N-$CH_3$), 2.25 (s, 6H, $N(CH_3)_2$), 2.1-2.7 (m, 5H, $CH_2$-a, $CH_2$-b und H-3'), 3.67 (bs, 1H, H-4'), 4.08 (q, 1H, $J_{5',6'}$ = 6.6 Hz, H-5'), 4.14 (bs, 1H, OH-9), 4.91 (s, 1H, H-10), 5.16 (m, 1H, H-7), 5.52 (bd, 1H, $J_{1',2'}$ = 2 Hz, H-1'), 7.34 (dd, 1H, $J_{1,3}$ = 1 Hz, $J_{2,3}$ = 8.4 Hz, H-3), 7.75 (t, 1H, $J_{1,2}$ = $J_{2,3}$ = 8 Hz, H-2), 7.90 (dd, 1H, $J_{1,2}$ = 7.6 Hz, $J_{1,3}$ = 1 Hz, H-1), 12.16 (bs, 1H, OH-4), 12.85 (bs, 1H, OH-6), 13.63 (bs, 1H, OH-11).

Beispiel 3:

7-0-(3'-N-(N,N'-Dimethyl-ethylendiaminoethyl)-3'-N-methyl-α-L-daunosaminyl)-β-rhodomycinon (Verbindung 3)

Zu einer Lösung von Verbindung 1 (50 mg = 0,084 mmol) in Ethanol (5 ml) wurde N,N'-Dimethyl-ethylendiamin zugetropft, bis eine kräftige Blaufärbung auftrat, und nachfolgend 16 h bei Raumtemperatur im Dunkeln gerührt. Dann wurde am Rotationsverdampfer zur Trockne eingeengt, das Reaktionsgemisch über 5 g Kieselgel im Laufmittelgemisch A getrennt ($R_F$ 0,17) und die gesammelten Fraktionen analog Beispiel 2 aufgearbeitet.
Ausbeute 23 mg (0,036 mmol) = 43 %
MS-FAB $(M+H^+)$ m/e = 644
$^1H$ NMR (300 MHz, $CDCl_3$) $\delta$ 1.12 (t, 3H, $J_{13,14}$ = 7.4 Hz, Me-14), 1.40 (d, 3H, $J_{5',6'}$ = 6.4 Hz, Me-6'), 1.6-1.9 (m, 4H, $CH_2$-2' und $CH_2$-13), 2.10 (dd, 1H, $J_{7,8a}$ = 4 Hz, $J_{8a,8b}$ = 15 Hz, H-8a), 2.20 (s, 3H, N-$CH_3$), 2.25 (s, 3H, N-$CH_3$), 2.25 (d, 1H, $J_{8a,8b}$ = 15 Hz, H-8b), 2.55 (s, 3H, N-$CH_3$), 2.34-2.72 (m, $CH_2$-a, $CH_2$-b, $CH_2$-c, $CH_2$d), 3.69 (bs, 1H, H-4'), 4.08 (q, 1H, $J_{5',6'}$ = 6.9 Hz, H-5'), 4.90 (s, 1H, H-10), 5.15 (m, 1H, H-7), 5.51 (bs, 1H, H-1'), 7.29 (d, 1H, $J_{2,3}$ = 8.3 HZ, H-3), 7.71 (t, 1H, $J_{1,2}$ = $J_{2,3}$ = 8 Hz, H-2), 7.88 (d, 1H, $J_{1,2}$ = 7.5 Hz, H-1).

Beispiel 4:

7-0-(3'-N-Histaminoethyl-3'-N-methyl-α-L-daunosaminyl)-β-rhodomycinon (Verbindung 4)

Eine Lösung von Verbindung 1 (50 mg = 0,084 mmol) in Dimethylformamid (6 ml) wurde mit festem Histamin versetzt, bis Farbumschlag nach blau erfolgte, und 20 h bei Raumtemperatur im Dunkeln gerührt. Dann wurde am Rotationsverdampfer zur Trockne eingeengt, das Reaktionsgemisch über 2 g Kieselgel im Laufmittelgemisch A getrennt ($R_F$ 0,26) und die gesammelten Fraktionen analog Beispiel 2 aufgearbeitet.
Ausbeute: 12 mg (0,018 mmol) = 21 %
MS-FAB $(M+H^+)$ m/e = 667
$^1H$ NMR (300 MHz, $CDCl_3/D_6$-DMSO (3/1)) $\delta$ 1.09 (t, 3H, $J_{13,14}$ = 7.4 Hz, Me-14), 1.33 (d, 3H, $J_{5',6'}$ = 6.4 Hz, Me-6'), 1.7-1.9 (m, 4H, $CH_2$-2' und $CH_2$-13), 2.17 (bs, 2H, $CH_2$-8), 2.19 (s, 3H, N-$CH_3$), 2.42-2.96 (m, 9H, $CH_2$-a, $CH_2$-b, $CH_2$-c, $CH_2$-d und H-3'), 3.68 (bs, 1H, H-4'), 3.97 (bs, 1H, OH-9), 4.07 (q, 1H, $J_{5',6'}$ = 6.7 Hz, H-5'), 4.78 (s, 1H, H-10), 5.11 (m, 1H, H-7), 5.49 (bs, 1H, H-1'), 6.74 (s, 1H, H-e), 7.27 (d, 1H, $J_{2,3}$ = 8.2 Hz, H-3), 7.47 (s, 1H, H-f), 7.69 (t, 1H, $J_{1,2}$ = $J_{2,3}$ = 8 Hz, H-2), 1.82 (d, 1H, $J_{1,2}$ = 7.4 Hz, H-1).

Beispiel 5:

7-0-(3'-N-Acetoxyethyl-3'-N-methyl-α-L-daunosaminyl)-β-rhodomycinon (Verbindung 5)

Die Umsetzung erfolgte analog Beispiel 1, wobei jedoch statt 1 h 6 h gerührt wurde. Während dieser Zeit wird das Chloratom der zunächst gebildeten Verbindung 1 durch Acetat substituiert. Die chromatographische Reinigung erfolgte im Laufmittelgemisch C ($R_F$ 0.65).
Ausbeute: 43 %
MS-FAB ($M+H^+$) m/e = 616
$^1$H NMR (300 MHz, $CDCl_3$) δ, 1.12 (t, 3H, $J_{13,14}$ = 7.4 Hz, Me-14), 1.41 (d, 3H, $J_{5',6'}$ = 6.6 Hz, Me-6'), 1.7-1.9 (m, 4H, $CH_2$-2' und $CH_2$-13), 1.99 (s, 3H, acetyl-$CH_3$), 2.12 (dd, 1H, $J_{7,8a}$ = 4 Hz, $J_{8a,8b}$ = 15 Hz, H-8a), 2.25 (d, 1H, $J_{8a,8b}$ = 15 Hz, H-8b), 2.26 (s, 3H, N-$CH_3$), 2.5-2.7 (m, 2H, H-a und H-3'), 2.8-2.9 (m, 1H, H-a'), 3.66 (bs, 1H, H-4'), 4.04-4.12 (m, 4H, OH-9 (s), H-5' (q) und $CH_2$-b (t)), 4.90 (s, 1H, H-10), 5.16 (m, 1H, H-7), 5.52 (bs, 1H, H-1'), 7.35 (dd, 1H, $J_{1,3}$ = 1 Hz, $J_{2,3}$ = 8.4 Hz, H-3), 7.73 (t, 1H, $J_{1,2}$ = $J_{2,3}$ = 8 Hz, H-2), 7.90 (dd, 1H, $J_{1,2}$ = 7.4 Hz, $J_{1,3}$ = 1 Hz, H-1).

Zytotoxizität von Verbindungen der Formel I gegen L1210-Leukämiezellen der Maus in vitro

Verfahrensweise zur Ermittlung der Koloniebildung von L1210 Leukämiezellen in Soft-Agar

500 Leukämiezellen pro Platte wurden mit unterschiedlichen Konzentrationen der Testsubstanz 1 Stunde bei 37° C inkubiert. Anschließend wurden die Zellen zweimal mit McCoy5A-Medium gewaschen und schließlich in Petrischalen nach Zugabe von 0,3 % Agar ausgegossen. Kontrollen wurden lediglich mit frischem Medium inkubiert. Anstelle der einstündigen Inkubation wurden in manchen Fällen unterschiedliche Konzentrationen und Testsubstanzen der oberen Agarschicht zugemischt, um so eine kontinuierliche Exposition der Zellen über die gesamte Inkubationszeit zu erreichen. Nach Erstarren des Agars wurden die Platten im Brutschrank 7 Tage bei 37° C inkubiert (5 Vol.-% $CO_2$, 95 % relative Luftfeuchtigkeit). Anschließend wurde die Zahl der entstandenen Kolonien mit einem Durchmesser von mehr als 60 µm gezählt. Die Ergebnisse wurden angegeben als Koloniezahl in behandelten Agarplatten in Prozent der unbehandelten Kontrolle. Aus der so erhalte nen Dosiswirkungskurve wurde die $IC_{50}$ als Maß für die Wirksamkeit der Substanz ermittelt. Die Ergebnisse für die hier beschriebenen Verbindungen im Vergleich zu Adriamycin sind in Tabelle 1 zusammengefaßt.

**Tabelle 1: Zytotoxizität der hergestellten Verbindungen der Formel I gegen L1210 Leukämiezellen in vitro**

| Substanz No. (Beispiel) | $R^1$ | $R^2$ | Dauerinkubation $IC_{50}$ (µg/ml) | 1 h-Inkubation $IC_{50}$ (µg/ml) |
|---|---|---|---|---|
| 1 | H | Cl | 0.03 | 0.11 |
| 2 | H | $N(CH_3)_2$ | 0.033 | 0.12 |
| 3 | H | $N(CH_3)-CH^c_2-CH^d_2-NH(CH_3)$ | 0.1 | 0.6 |
| 4 | H | $NH-CH^c_2-CH^d_2-$ 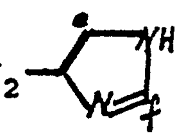 | 0.4 | 1.2 |
| 5 | H | $OCOCH_3$ | | |

## Ansprüche

1. Neue zytostatisch wirksame Anthracyclinderivate der Formel I

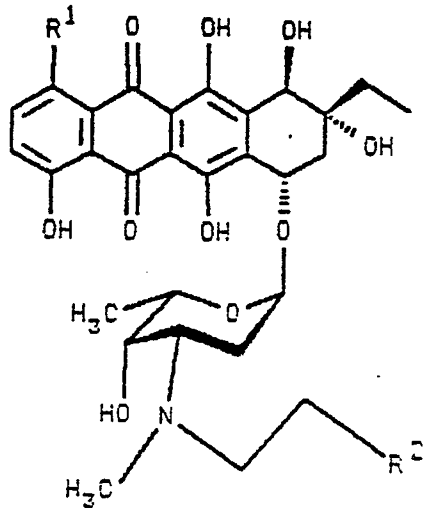

und deren physiologisch unbedenklichen Salze , wobei

$R^1$ Wasserstoff oder eine Hydroxygruppe und

$R^2$ Cl, Br, $NR^aR^b$, $NR^a$-$(CH_2)_n$-$NR^aR^b$, $NR^a$-$NR^a$-CO-$(CH_2)_y$-CO-$NR^a$-$NR^aR^b$ mit n = 0-12, bevorzugt mit n = 0 oder n = 2-5, und y = 0-10, bevorzugt mit y = 0-5 ist, wobei $R^a$ und $R^b$ jeweils Wasserstoff, $C_1$-$C_4$-Alkyl oder Benzyl sein können, oder

$R^2$ $NR^a$-$CHR^3$-$CH_2$-$R^4$ ist,

wobei $R^a$ die genannte Bedeutung hat und $R^3$ Wasserstoff oder eine Carboxylgruppe und $R^4$ den Rest einer L-Aminosäure oder eines biogenen Amins darstellt, oder

$R^2$ $OCOR^X$ ist, wobei $R^x$ $C_1$-$C_6$-Alkyl, verzweigt oder unverzweigt, Phenyl oder Benzyl darstellt.

2. Anthracyclinderivate nach Anspruch 1,
**dadurch gekennzeichnet,**
daß $R^2$ Cl oder Br, bevorzugt jedoch Cl ist.

3. Anthracyclinderivate nach Anspruch 1,
**dadurch gekennzeichnet,**
daß $R^2$ $NR^aR^b$ ist.

4. Anthracyclinderivate nach Anspruch 1,
**dadurch gekennzeichnet,**
daß $R^2$ $NR^a(-CH_2)_n$-$NR^aR^b$ ist.

5. Anthracyclinderivate nach Anspruch 1,
**dadurch gekennzeichnet,**
daß $R^2$ $NR^a$-$NR^a$-CO-$(CH_2)_y$-CO-$NR^a$-$NR^aR^b$ ist.

6. Anthracyclinderivate nach Anspruch 1,
**dadurch gekennzeichnet,**
daß $R^2$ $NR^a$-$CHR^3$-$CH_2$-$R^4$ ist, wobei die Verbindungen, in welchen $R^3$ Wasserstoff ist, bevorzugt sind.

7. Anthracyclinderivate nach Anspruch 6,
**dadurch gekennzeichnet,**
daß $R^4$ den Rest einer aromatischen L-Aminosäure oder eines aromatischen biogenen Amins darstellt.

8. Anthracyclinderivate nach Anspruch 7,
**dadurch gekennzeichnet,**
daß $R^4$ den Rest des biogenen Amins Tyramin, Tryptamin, Histamin, Dopamin oder 5-Hydroxydopamin und bevorzugt den Rest des biogenen Amins Histamin darstellt.

9. Anthracyclinderivate nach Anspruch 1,
**dadurch gekennzeichnet,**
daß $R^2$ $OCOR^X$ ist, wobei $R^x$ = $CH_3$ bevorzugt ist.

10. Verfahren zur Herstellung von Anthracyclinderivaten nach Anspruch 1,
**dadurch gekennzeichnet,**
daß man eine Verbindung der Formel II,

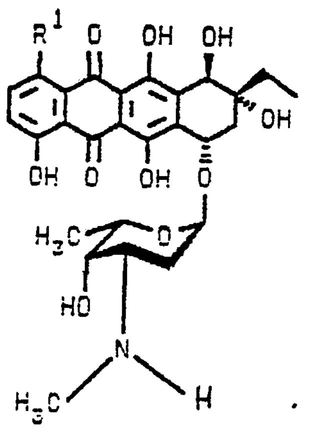

II

in welcher $R^1$ die genannte Bedeutung hat, durch Umsetzung mit Chloracetaldehyd oder Bromacetaldehyd in Gegenwart von Natriumcyanoborhydrid in eine Verbindung der Formel I überführt, in welcher $R^1$ die genannte Bedeutung hat und $R^2$ = Cl oder Br ist, worauf man diese Verbindung mit einer Verbindung der Formel $HNR^aR^b$ oder $HR^aN-(CH_2)_n-NR^aR^b$ oder $HNR^a-NR^a-CO-(CH_2)_y-CO-NR^a-NR^aR^b$, worin n, $R^a$, $R^b$ und Y die genannte Bedeutung haben, oder mit einem biogenen Amin oder einer Aminosäure oder mit einer Verbindung der Formel $MeOCOR^x$, wobei $R^x$ $C_1$-$C_6$-Alkyl, verzweigt oder unverzweigt, Phenyl oder Benzyl ist und Me ein Alkaliion darstellt, in einem geeigneten Lösungsmittel, bevorzugt Dimethylformamid, bei einer Temperatur zwischen 0°C und der Siedetemperatur des Lösungsmittels zur Reaktion bringt und die so hergestellte Verbindung der Formel I isoliert und aufreinigt.

11. Anthracyclinderivat nach Anspruch 1 als Arzneimittel.

Patentanspruch für folgende Vertragsstaaten: ES, GR

1. Verfahren zur Herstellung von neuen zytostatisch wirksamen Anthracyclinderivaten der Formel I

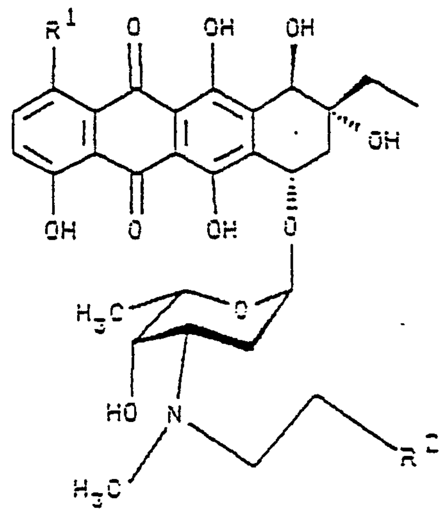

und deren phxysiologisch unbedenklichen Salze, wobei

$R^1$ Wasserstoff oder eine Hydroxygruppe und

$R^2$ Cl, Br, $NR^aR^b$, $NR^a-(CH_2)_n-NR^aR^n$, $NR^a-NR^a-CO-(CH_2)_y-CO-NR^a-NR^aR^b$ mit n = 0-12, bevorzugt mit n = 0 oder n = 2-5, und y = 0-10, bevorzugt y = 0-5 ist, wobei $R^a$ und $R^b$ jeweils Wasserstoff, $C_1$-$C_4$-Alkyl oder Benzyl sein können, oder

$R^2$ $NR^a-CHR^3-CH_2-R^4$ ist,

wobei $R^a$ die genannte Bedeutung hat und $R^3$ den Rest einer L-Aminosäure oder eines biogenen Amins darstellt, oder

$R^2$ $OCOR^x$ ist, wobei $R^x$ $C_1$-$C_6$-Alkyl, verzweigt oder unverzweigt, Phenyl oder Benzyl darstellt,

dadurch gekennzeichnet, daß man eine Verbindung der Formel II,

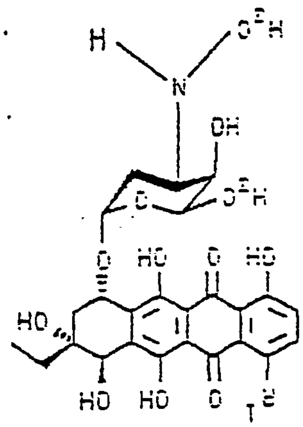

in welcher $R^1$ die genannte Bedeutung hat, durch Umsetzung mit Chloracetaldehyd oder Bromacetaldehyd in Gegenwart von Natriumcyanoborhydrid in eine Verbindung der Formel I überführt, in welcher $R^1$ die genannte Bedeutung hat und $R^2$ = Cl oder Br ist, worauf man diese Verbindung mit einer Verbindung der Formel $HNR^aR^b$ oder $HR^aN\text{-}(CH_2)_n\text{-}NR^aR^b$ oder $HNR^aNR^a\text{-}CO\text{-}(CH_2)_y\text{-}CO\text{-}NR^a\text{-}NR^aR^b$, worin n, $R^a$, $R^b$ und Y die genannte Bedeutung haben, oder mit einem biogenen Amin oder einer Aminosäure oder mit einer Verbindung der Formel $MeOCOR^x$, wobei $R^x$ $C_1$-$C_6$-Alkyl, verzweigt oder unverzweigt, Phenyl oder Benzyl ist und Me ein Alkaliion darstellt, in einem geeigneten Lösungsmittel, bevorzugt Dimethylformamid, bei einer Temperatur zwischen 0°C und der Siedetemperatur des Lösungsmittels zur Reaktion bringt und die so hergestellte Verbindung der Formel I isoliert und aufreinigt.